# EUROPEAN PATENT SPECIFICATION

(11) **EP 2 892 571 B1**
(45) Date of publication and mention of the grant of the patent: **12.10.2016**
(21) Application number: 13779372.5
(22) Date of filing: 14.08.2013
(51) Int. Cl.: A61L 2/07, A61L 11/00, B02C 19/10, B09B 3/00

(54) **MACHINE AND METHOD FOR THE TREATMENT OF HAZARDOUS MEDICAL WASTE**
MASCHINE UND VERFAHREN ZUR BEHANDLUNG VON GEFÄHRLICHEN MEDIZINISCHEN ABFÄLLEN
MACHINE ET PROCÉDÉ POUR LE TRAITEMENT DE DÉCHETS MÉDICAUX DANGEREUX

(30) Priority: 04.09.2012 IT BO20120467
(43) Date of publication of application: 15.07.2015
(73) Proprietor: Angelantoni Life Science S.r.l., 06056 Massa Martana (PG) (IT)
(72) Inventor: PARENTINI, Ignazio, I-19121 La Spezia (IT)
(74) Representative: Papa, Elisabetta
(86) International application number: PCT/IB2013/056639
(87) International publication number: WO 2014/037831

(56) References cited:
- WO-A1-2007/014312
- WO-A1-2012/049482
- WO-A2-2007/079968
- WO-A2-2011/092509
- US-A- 4 578 185

## Description

The present invention relates to a machine and to a method for the treatment of hazardous medical waste.

### PRIOR ART

Medical waste which is generated in health centres (public hospitals, private hospitals, consulting rooms, medical laboratories, research and diagnostic laboratories, centres for research on animals, veterinary centres, mortuary and autopsy centres, blood banks, etc.) can be of different nature; in particular among the medical waste there is also the infected and pathological waste (*"Bio Hazard Waste"*), which has got in touch with biological liquids and can be carriers of infections and diseases. The infected and pathological waste is classified as dangerous waste and it must be treated with systems leading to the inactivation thereof in order to secure operators, sanitary environments and the environment, in general.

Nowadays several technological solutions are known to lower the bacterial and viral charge of this kind of waste which distinguish based upon the system complexity as well as the sanitization type and the adopted shredding modes: incinerators, systems with ionizing radiations, sterilizing systems with disinfectant fluids or with vapour.

However, none of these systems nowadays at the same time is sufficiently safe and economic in treating this particular waste category.

WO 2011/092509 discloses a rotary autoclave for sterilising waste, wherein the autoclave is downwardly inclined towards its discharge end and has a door at the discharge end; and injecting steam through said door into said autoclave. to treat the load.

WO9112889, US2001016181, US5346142 and WO9217275, for example, describe rather complex systems providing a plurality of sanitization and shredding phases, often intercalated one to the other one, wherein usually the sanitization agent is a liquid chemical agent and the use thereof, inside the plant, produces waste liquid flows to be subjected to additional disposal. A structurally simpler solution is described in US5087420, still operating with liquid disinfectant means. These systems are poorly effective and furthermore, by using sterilizing liquids for treating the waste, the use thereof results definitely expensive (due to the cost of the sterilizing liquids) and complex (since one has to feed huge quantities of sterilizing liquids to the machine, which will have then to be subsequently disposed).

In order to avoid using sterilizing liquids, other machines have been proposed, in particular operating with vapour sanitization systems, according to what suggested by the most recent guidelines of WHO (World Health Organization) as to treatment of medical dangerous waste.

Some of these apparatuses implement in combined way the sterilisation and the shredding of waste; the patent documents CN201806976, CN201692370, US2006255191 and JP2008054841, describe examples of apparatuses of this type. The efficiency of these machines in terms of sanitization is never the optimum one, so that the same patent texts report the need to make these preliminary treatments to be followed by subsequent and additional sterilization phases.

Apparatuses for the sanification of medical waste are currently available on the market operating in two distinct phase for shredding and vapour sanitization.

In cases wherein the vapour sanitization precedes the waste shredding, as described for example in CN201244221 and WO2004071667, the sterilization is not optimum and the material often remains infected as the vapour does not succeed in reaching all points and surfaces to be sterilized, as in case of the products wrapped in protective material or the closed containers.

In case the systems perform at first shredding and then sterilization of the infected material, the shredder remains continuously infected and this involves the possibility and the development of crossed contaminations and a higher bacterial charge with consequent serious handling risks for the technical and nursing personnel responsible for these operations.

Therefore it is still felt the need for an apparatus which is able in a structurally simple and economic way to treat medical dangerous waste, with safety and efficiency, by overcoming all limits illustrated sofar with reference to the known apparatuses and available on the market of the type of those described in the patent applications US2006255191A1 and CN201806976A, treating shred waste by means of water vapour. However, these known machines too are not particularly effective nor particularly efficient as they do not succeed (unless with extremely long treatment time) in guaranteeing a complete sterilization of waste.

### SUMMARY OF THE INVENTION

The object of the present invention is to provide a machine and a method for the treatment of hazardous medical waste which are free from the above described drawbacks and at the same time are easy and economic to be implemented.

According to the present invention a machine and a method for the treatment of hazardous medical waste are provided, according to what claimed by the enclosed claims.

### BRIEF DESCRIPTION OF THE DRAWINGS

The present invention will be now described with reference to the enclosed drawings, which illustrate a not limitative embodiment example thereof, wherein:
- figure 1 is a front, schematic view, with removed portions for clarity, of a machine for the treatment of medical dangerous waste implemented according to the present invention; and
- figure 2 is a side, schematic view, with removed portions for clarity, of the machine of figure 1.

### PREFERRED EMBODIMENTS OF THE INVENTION

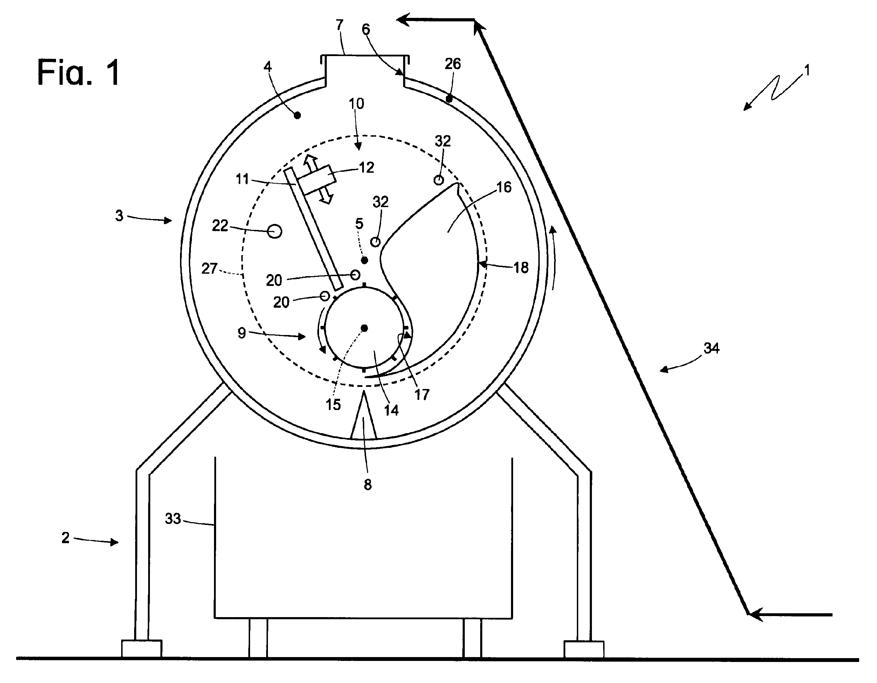

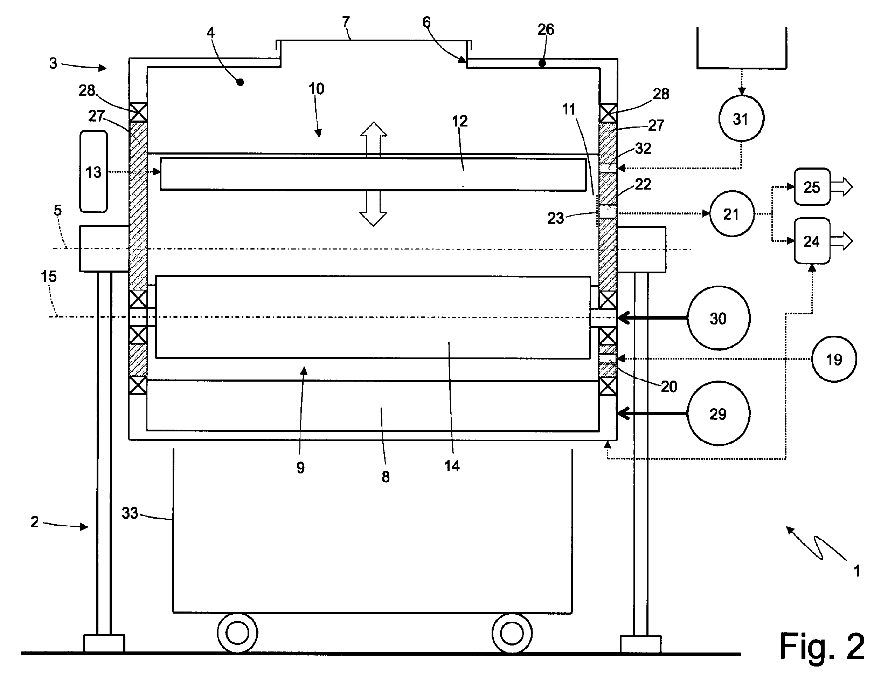

In figures 1 and 2, a machine for the treatment of medical dangerous waste is designated as a whole with number 1.

The machine 1 comprises a frame 2 resting upon the floor and supporting a cylindrical and sealed containing body 3 inside thereof a treatment chamber 4 is defined; the containing body 3 is mounted so as to rotate around a horizontal rotation axis 5 and it is preferably made of stainless steel AISI 316Ti which is mirror polished inside.

The containing body 3 is provided with an inlet/outlet opening 6 which is obtained through a side wall of the containing body 3 and it is provided with a mobile lid 7; preferably, the lid 7 is hinged to the containing body 3 and it is provided with a pneumatic actuator opening/closing automatically the lid 7 by guaranteeing the hermetic sealing of the lid in the closing position.

The containing body 3 is provided with a pushing element 8 which is arranged inside the treatment chamber 4, it projects from an inner wall of the containing body 3, and it is integral to the containing body 3 so as to rotate around the rotation axis together with the containing body 3 in order to push the waste arranged inside the treatment chamber 4 from the bottom to the top.

The machine 1 comprises a shredding device 9 which is arranged inside the treatment chamber 4 in a fixed position so that the containing body 3 rotates around the shredding device 9. Furthermore, the machine 1 comprises a conveying device 10 which is arranged inside the treatment chamber 4 and above the shredding device 9 in a fixed position so that the containing body 3 rotates around the conveying device 10. The function of the conveying device 10 is to convey the waste falling by gravity from the top to the bottom in the shredding device 9. When the containing body 3 rotates around the rotation axis 5 (counterclockwise as illustrated in figure 1) the pushing element 8 cyclically pushes the waste arranged in the treatment chamber 4 from the bottom (that is from the area arranged below the shredding device 9) to the top (that is above the conveying device 10); in this way the rotation of the containing body 3 determines a continuous passage of the waste through the shredding device 9.

The conveying device 10 has a "funnel-like" shape which converges on the shredding device 9 and comprises a flat chute 11 which is arranged in an inclined manner and ends close to the shredding device 9. Furthermore, the conveying device 10 comprises a pressing element 12 which is mounted so as to slide along the chute 11 and an actuating device 13 (preferably a double-effect pneumatic actuator) which is mechanically connected to the pressing element 12 so as to cause the pressing element 12 to move with a linear reciprocating motion along the chute 11 (that is parallelly to the chute 11). In other words, upon use the pressing element 12 slides forwards and backwards (that is by approaching and moving away from the shredding device 9) along the chute 11 in order to push cyclically the waste inside the shredding device 9.

The shredding device 9 comprises a single shredding drum 14 which is mounted so as to rotate around a rotation axis 15 parallel to the rotation axis 5 and it is provided with blades that project from the periphery of the shredding drum 14 itself. The rotating motion of the shredding drum 14 around its own axis 15 is independent from the rotation motion of the body 3, but the two motions will be synchronized in order to guarantee fluidity and continuity of the process. Furthermore, the shredding device 9 comprises a fixed countering body 16 which is arranged next to the shredding body 14 and it has a curved countering wall 17 which cooperates with the shredding drum 14 and it is arranged around a portion of the shredding drum 14 itself. According to a preferred embodiment, 9, the countering body 16 in its cross section has a "comma-like" shape (which can be well seen in figure 1) and on the side facing the shredding drum 14 it has the curved countering wall 17 and, on the opposite side, a curved accompanying wall 18 which, on the lower side, is connected to the countering wall 17. The accompanying wall 18, together with the inner wall of the containing body 3, on the right side of the treatment chamber 4 defines an ascending channel along thereof the waste is pushed from the bottom to the top due to the action of the pushing element 8. It is important noting that an upper portion of the countering body 16 cooperates with the chute 11 in order to define a "funnel-like path" which conveys the waste falling by gravity from the top to the bottom on the shredding device 9.

According to a different and not illustrated embodiment, the shredding device 9 comprises two or more shredding drums 14 arranged side-by-side therebetween and cooperating. According to an additional different and not illustrated embodiment, the fixed countering body 16 is replaced by one or more shredding drums 14.

The machine 1 comprises a vapour feeding device 19 (schematically illustrated in figure 2) which is apt to feed vapour under pressure inside the treatment chamber 4 through a plurality of inlet openings 20 (only a portion thereof is schematically illustrated in the enclosed figures); preferably, the inlet openings 20 are concentrated near the shredding device 9 and/or the conveying device 10 in order to better distribute the vapour among the waste during the shredding thereof. According to a possible embodiment, the inlet openings 20 are obtained through a (internally hollow) shaft of the shredding drum 14. The vapour feeding device 19 can comprise inside thereof a vapour generator or it can receive the vapour from an outer vapour generator.

Furthermore, the machine 1 comprises a vacuum device 21 (provided with a dry pump compatible with vapour) which is pneumatically connected to the treatment chamber 4 in order to implement the vacuum inside the treatment chamber 4 itself; in particular, the vacuum device 21 communicates with the treatment chamber 4 through at least an outlet opening 22 which is provided with a mechanical filter 23 with very narrow meshes (schematically illustrated in figure 2 only) which prevents solid residues with significant size from being discharged. A bacteriological filter 24 (usually of "HEPA" type), which can be connected to an outlet of the vacuum device 21 discharging into the atmosphere the gas sucked from the treatment chamber 4 is provided; preferably, the vapour feeding device 19 can be connected to the bacteriological filter 24 in order to sterilize the bacteriological filter 24 itself. Furthermore, a burner 25 is provided, which can be connected to the outlet of the vacuum device 21 which discharges into the atmosphere the gas sucked from the treatment chamber 4 and it is apt to heat the gas at a predetermined temperature for a predetermined time interval. Obviously, (not illustrated) electrovalves are provided adjusting the connections of the outlet of the vacuum device 21 to the bacteriological filter 24 and to the burner 25 and adjusting the connection of the bacteriological filter 24 to the vapour feeding device 19.

According to a preferred embodiment, a cylindrical side wall of the containing body 3 can be heated; preferably, the cylindrical side wall of the containing body 3 has a hollow space 26 inside thereof vapour coming from the vapour feeding device 19 can be fed. Alternatively, electrical thermoresistances are housed in the hollow space 26

Two fixed flanges 27 with circular shape are provided which are supported by the frame 2, are coaxial to the rotation axis 5 and are arranged on opposite sides of the treatment chamber 4 so as to define two end closures of the treatment chamber 4 itself. The two flanges 27 support two corresponding annular bearings 28 of the containing body 3 which are mounted along the outer edge of the flanges 27 themselves. Furthermore, the two flanges 27 support the shredding device 9 and the conveying device 10. Preferably, the inlet openings 20 of the vapour feeding device 19 and the outlet opening 22 of the vacuum device 21 are obtained through a flange 27.

The frame 2 supports an actuating device 29 (typically an electric motor) so as to rotate the containing body 3 around the rotation axis 5 (usually the rotation of the containing body 3 is slow and it takes place at a speed of about 0.25-4 revolutions per minute) and an actuating device 30 (typically an electric motor) to rotate the shredding drum 14 around the rotation axis 15 (usually the rotation of the shredding drum 14 is relatively fast and it takes place at a speed of about 15-60 revolutions per minute).

According to a preferred embodiment, a washing water feeding device 31 is provided which is apt to feed washing water inside the treatment chamber 4 through inlet openings 32 obtained in a flange 27.

As said previously, the frame 2 rests on the floor and it rigidly supports the two fixed flanges 27; furthermore, the frame 2 defines a discharge area, under the containing body 3, which is suited to house a discharge container 33 for the treated waste falling by gravity into the discharge container 33 through the inlet/outlet opening 6 which is arranged in a lower position (that is arranged in a position diametrically opposed to the upper position illustrated in figures 1 and 2).

At last, the machine 1 comprises a loading conveyor 34 (schematically illustrated in figure 1) which is arranged next to the containing body 3 and it is apt to lift the waste to be treated above the containing body 3 so as to cause the waste to fall, by gravity, into the treatment chamber 4 and through the inlet/outlet opening 6 which is arranged in an upper position (that is arranged in the position illustrated in figures 1 and 2).

The operation of the machine 1 for the treatment of medical dangerous waste is described herebelow.

Firstly, the containing body 3 is arranged in the position illustrated in figures 1 and 2 wherein the inlet/outlet opening 6 is in the upper position and therefore the lid 7 is opened; at this point, the loading conveyor 34 is activated to lift the waste (typically contained in sealed sacks with red colour) above the containing body 3 and then to make to fall the waste by gravity into the treatment chamber 4 through the inlet/outlet opening 6. During the filling-in of the treatment chamber 4 with the waste to be treated, the shredding device 9 and the associated conveying device 10 are activated (whereas the containing body 3 obviously remains still) so as to compact the waste and then favour the correct filling-in of the treatment chamber 4.

Once ended the filling-in of the containing body 3, the loading conveyor 34 is stopped and the lid 7 is closed to hermetically seal the treatment chamber 4. At this point the waste treatment cycle can start.

Initially, the waste is subjected to a repeated shredding by activating the shredding device 9 and the associated conveying device 10 whereas the containing body 3 is rotated around the rotation axis 5; in this way, and as already described previously, the pushing element 8 cyclically pushes the waste present in the treatment chamber 4 from the bottom (that is from the area arranged under the shredding device 9) to the top (that is above the conveying device 10) so as to determine a continuous passage of the waste through the shredding device 9.

At the end of the shredding (which generally is prolonged for few minutes) the vacuum device 21 is activated so as to generate and maintain the vacuum inside the treatment chamber 4 for a time interval T1 (in the order of 1-3 minutes).

At the end of the time interval T1 the vacuum device 21 is de-activated in order to interrupt the generation of the vacuum and therefore the vapour feeding device 19 is activated for feeding vapour under pressure into the treatment chamber 4 for a time interval T2 (in the order of 1-3 minutes); indicatively, the vapour is fed into the treatment chamber 4 at a temperature of 130-150 °C and at a pressure of 3-5 bar.

At the end of the time interval T2 the vapour feeding device 19 is de-activated to interrupt the feeding of vapour under pressure into the treatment chamber 4 and therefore the vacuum device 21 is again activated for generating and maintaining again the vacuum inside the treatment chamber 4 for a time interval T3 (in the order of 1-3 minutes).

According to a possible embodiment the steps for feeding vapour under pressure into the treatment chamber 4 for the time interval T2 and for generating the vacuum inside the treatment chamber 4 for the time interval T3 are repeated once in this order. Alternatively, the steps for feeding vapour under pressure into the treatment chamber 4 for the time interval T2 and for generating the vacuum inside the treatment chamber 4 for the time interval T3 are cyclically repeated in this order for a predetermined number of times (for example 2-4 times).

At the end of the last time interval T3, the treatment cycle is ended and therefore the sterilized waste can be discharged from the treatment chamber 4.

Before discharging the waste from the treatment chamber 4, that is before opening the treatment chamber 4 by opening the lid 7, it is necessary to bring back the treatment chamber 4 to the atmospheric pressure (at the end of the time interval T3 the treatment chamber 4 is under depression, that is vacuum sealed, due to the sucking action performed by the vacuum device 21); to this purpose, air is fed into the treatment chamber 4 by means of an air feeding device (which, usually, comprises an electro-actuated valve which is arranged through the wall of the treatment chamber 4). According to a possible embodiment, such air feeding device could comprise a bacteriological filter (similar to the bacteriological filter 24) and/or a burner (similar to the burner 25) which can be both activated or alternatively to sterilize the air which is fed into the treatment chamber 4.

According to a preferred embodiment, during the step of feeding vapour under pressure into the treatment chamber 4 and during the steps of generating the vacuum inside the treatment chamber 4 the shredding of waste by means of the shredding device 9 is performed continuously (obviously together with the rotation of the containing body 3 around the rotation axis 5 so as to permit a continuous crossing of the waste through the shredding device 9). In this way, the step of feeding vapour under pressure into the treatment chamber 4 is more effective as the vapour is able to reach all portions of the waste. Analogously, in this way even the steps of generating the vacuum inside the treatment chamber 4 are more effective as they succeed in eliminating all air bubbles which can remain trapped among the waste.

During the whole life of the above-described waste treatment cycle, a cylindrical side wall of the containing body 3 is constantly heated by feeding vapour into the hollow space 26. It is important that the cylindrical side wall of the containing body 3 is always hot during the step of feeding vapour into the treatment chamber 4 in order to avoid that the waste portions in contact with the inner wall of the containing body 3 can be colder than vapour (due to the effect of the heat exchange with the inner wall of the containing body 3) and thus they could "escape" at least partially the heating caused by vapour. Furthermore, it is important that the cylindrical side wall of the containing body 3 is always hot during the steps of generating the vacuum inside the treatment chamber 4 so as to determine the evaporation of possible residual liquids existing in the treatment chamber 4 (the residual liquids tend to go downwards and thus to collect at the bottom of the containing body 3 in contact with the inner wall of the containing body 3 itself) and therefore it allows the evacuation of such residual liquids (under the form of vapour) through the vacuum device 21.

When the vacuum device 21 is activated for the first time (that is during the time interval T1), the gas sucked from the treatment chamber 4 is discharged into the atmosphere by passing through the bacteriological filter 24; on the contrary, when the vacuum device 21 is activated subsequently to the first time (that is during the time interval T3), the gas sucked from the treatment chamber 4 is discharged into the atmosphere by passing through the burner 25 which, indicatively, heats the gas at about 300-400 °C for 10-30 seconds (the passage through the burner 25 would be not strictly necessary, as the inside of the treatment chamber 4 has been already sterilized by vapour, but however it is an additional precaution to have a redundant safety).

Once ended the treatment cycle, the containing body 3 is arranged in a position diametrically opposed to the position illustrated in figures 1 and 2 wherein the inlet/outlet opening 6 is in the lower position and therefore the lid 7 is opened; in this way, the treated and dry waste (which by now is almost under the powder form) outgoes by gravity from the treatment chamber 4 through the inlet/outlet opening 6 and it collects in the underneath discharge container 33. In case, the containing body 3 could be vibrate with small oscillations around the rotation axis 5 so as to permit the outgoing by gravity of the treated and dry waste through the inlet/outlet opening 6.

The discharge container 33 could comprise an automatic filling device sucking the treated and dry waste from the bottom of the discharge container 33 so as to transfer the treated and dry waste itself in collecting small sacks.

It is important underlying that the machine 1 can provide in an autonomous and automatic way the periodic sterilization (for example each certain number of treatment cycles) of the bacteriological filter 24 by making to pass through the bacteriological filter 24 itself the vapour coming from the vapour feeding device 19; obviously such sterilization takes place when the bacteriological filter 24 is not used.

Furthermore, it is important underlying that the machine 1 can provide in autonomous and automatic way the periodic washing of the treatment chamber 4 (for example at the end of each day); such washing (which obviously is performed outside one treatment cycle when the treatment chamber 4 is empty) provides to feed into the treatment chamber 4 washing water (in case added with detergent) by means of the washing water feeding device 31, to heat the containing body 3 at the sterilization temperature whereas the containing body 3 is rotated and the shredding device 9 is activated, to dry the treatment chamber 4 by making the washing water to evaporate and, at last, to eliminate the vapour formed by the washing water by means of sucking implemented by the vacuum device 21.

All portions in contact with vapour and with waste are preferably provided with surface coating so as to resist to the mechanical action and to the chemical corrosion which develop due to the acids existing in the waste (this coating guarantees the anti-adherence too).

The machine 1 comprises a control electronic unit which supervises the operation of all components and it is provided with a PLC; preferably a control display of "touch-screen" type is provided, which is connected to the control electronic unit and plays the function of HMI (*"Human Machine Interface"*) device. On the display several pages are displayed wherein, each time, for programming and handling the machine there will appear: a main menu, a library of cycles, the cycle parameters, the data related to the load (operator, lot), the plant general conditions for the cycle beginning, a real time diagramme of variables of sterilization process, the process control, the state of the shredding device 9, the rotation state of the containing body 3, the programmed maintenance, a remote control, the instructions for the maintenance, the alarms, a date control, the display of temperatures and F0, various messages (state condition of doors, temperatures, pressures, vacuum, etc.).

Preferably, the control electronic unit is connected to an alphanumerical printer, installed on a control panel, for the paper documentation of messages, parameters and regular progress of the cycles.

The above-described machine 1 has several advantages.

First of all, the above described machine 1 allows treating medical dangerous waste in an extremely effective and efficient way and in the full environment respect (no polluting substance is produced during the treatment cycle). In particular, the above-described machine 1 guarantees to obtain the complete sterilization of medical dangerous waste in a reduced treatment time (in the order of 10-20 minutes); in fact, by using the vapour as sterilizing agent in combination with the vacuum it is possible obtaining a microbial reduction of 1,000,000:1 (sterilization level). Furthermore, the extreme shredding and the continuous mixing of waste allows the vapour and the vacuum to act in a very effective way.

In other words, the operations of sterilization (with cycles of vacuum and vapour inletting), repeated shredding and waste movement take place contemporaneously in the treatment chamber 4 of the machine by obtaining for this reason the following advantages: optimization of the whole process, absence of need for handling the infected waste, optimization of the vapour treatment and maintenance of the machine under safety conditions (in particular against the biological risk) without particular disinfection treatments.

Furthermore, the above described machine 1 allows obtaining dry and extremely compacted waste (the volume reduction can overcome 90%); the extreme compaction is obtained by making the waste to pass several times through the shredding device 9.

It is important underlying that no discharge of liquids ever takes place, as the water is always ejected under the form of vapour (after passage through the bacteriological filter 24 or the burner 25).

The above described machine 1 can be fully automatized, that is the whole treatment cycle takes place in a wholly automatic way; in this way the risks for the operators are minimized and above all the presence of expert operators, with high skills, is not required.

The above described machine 1 is easy and economic to be implemented, as it has an easy base structure.

At last, the above-described machine 1 is particularly robust and therefore it has a very long operating life even though it requires a modest and a little frequent periodic maintenance.

## Claims

1. A machine (1) for the treatment of medical hazard waste; the machine (1) comprises:
a cylindrical containing body (3), which is mounted so as to rotate around a first rotation axis (5) and inside which a treatment chamber (4) is defined;
a first actuating device (29), apt to rotate the containing body (3) around the first rotation axis (5);
an inlet/outlet opening (6), which is obtained through a lateral wall of the containing body (3) and is provided with a mobile lid (7); and
a vapour feeding device (19), which is apt to feed vapour under pressure into the treatment chamber (4);
the machine (1) is **characterised in that** it comprises:
at least a pushing element (8), which is arranged inside the treatment chamber (4), projects from an inner wall of the containing body (3), and is integral to the containing body (3) so as to rotate around the first rotation axis (5) together with the containing body (3), in order to push the waste arranged inside the treatment chamber (4) from the bottom to the top;
a shredding device (9), which is arranged inside the treatment chamber (4) in a fixed position, so that the containing body (3) rotates around the shredding device (9);
a conveying device (10), which is arranged inside the treatment chamber (4) and above the shredding device (9) in a fixed position, so that the containing body (3) rotates around the conveying device (10); and
a vacuum device (21), which is pneumatically connected to the treatment chamber (4), so as to create a vacuum inside the treatment chamber (4).

2. A machine (1) according to claim 1 and comprising a mechanical filter (23), which is coupled to an outlet opening (22) of the treatment chamber (4), which communicates with the vacuum device (21).

3. A machine (1) according to claim 1 or 2 and comprising at least one bacteriological filter (24), which can be connected to an outlet of the vacuum device (21), which discharges the gas sucked from the treatment chamber (4) into the atmosphere.

4. A machine (1) according to claim 3, wherein the vapour feeding device (19) can be connected to the bacteriological filter (24), so as to sterilize the bacteriological filter (24).

5. A machine (1) according to any of the preceding claims, comprising at least one burner (25), which can be connected to an outlet of the vacuum device (21), which discharges the gas sucked from the treatment chamber (4) into the atmosphere, and is apt to heat the gas at a predetermined temperature for a predetermined time interval.

6. A machine (1) according to any of the preceding claims, wherein the conveying device presents a "funnel" shape, which converges on the shredding device (9) and comprises a flat chute (11), which is arranged in an inclined manner and ends close to the shredding device (9).

7. A machine (1) according to claim 6, wherein the conveying device (10) comprises:
a pressing element (12), which is mounted so as to slide along the chute (11); and
a second actuating device (13), which is mechanically connected to the pressing element (12), so as to cause the pressing element (12) to move with a linear reciprocating motion parallel to the chute (11).

8. A machine (1) according to any of the preceding claims, wherein the shredding device (9) comprises:
a single shredding drum (14), which is mounted so as to rotate around a second rotation axis (15), which is parallel to the first rotation axis (5), and is provided with blades that project from the periphery of the shredding drum (14);
a third actuating device (30), apt to rotate the shredding drum (14) around the second rotation axis (15); and
a fixed countering body (16) having a curved countering wall (17) which cooperates with the shredding drum (14) and is arranged around a portion of the shredding drum (14).

9. A machine (1) according to claim 8, wherein the countering body (16) has a "comma" shape in its transverse section and presents the curved countering wall (17) on the side facing the shredding drum (14), and, on the opposite side, a curved accompanying wall (18), which, on the lower side, is connected to the countering wall (17) and defines, together with an inner wall of the containing body (3), an ascending channel along which the waste is pushed from the bottom to the top due to the action of the pushing element (8).

10. A machine (1) according to any of the preceding claims, wherein a lateral cylindrical wall of the containing body (3) is heatable.

11. A machine (1) according to claim 10, wherein the lateral cylindrical wall of the containing body (3) presents a hollow space (26), into which vapour coming from the vapour feeding device (19) can be fed.

12. A machine (1) according to any of the preceding claims comprising a washing water feeding device (31), which is apt to feed washing water into the treatment chamber (4).

13. A machine (1) according to any of the preceding claims, comprising:
two fixed flanges (27) with a circular shape, which are coaxial to the first rotation axis (5), are arranged on opposite sides of the treatment chamber (4) so as to define two end closures of the treatment chamber (4), support two corresponding annular bearings (28) of the containing body (3) that are mounted along the outer edge of the flanges (27), and support the shredding device (9) and the conveying device (10).

14. A machine (1) according to claim 13 and comprising a frame (2), which rests on the floor, rigidly supports the two fixed flanges (27), and defines, under the containing body (3), a discharges area, which is apt to house a discharge container (33) for the treated waste that falls, by gravity, into the discharge container (33) through the outlet/inlet opening (6), which is arranged in a lower position.

15. A machine (1) according to any of the preceding claims, comprising a loading conveyor (34), which is arranged next to the containing body (3) and is apt to lift the waste to be treated above the containing body (3), so as to cause it to fall, by gravity, into the treatment chamber (4) and through the inlet/outlet opening (6), which is arranged in an upper position.

16. A method for the treatment of hazardous medical waste; the method comprises the steps of:
feeding the waste into a treatment chamber (4), which is defined inside a cylindrical containing body (3), which is mounted so as to rotate around a rotation axis (5); and
shredding the waste by means of a shredding device (9), which is arranged inside the treatment chamber (4);
the method is **characterised in that** it comprises the further steps of:
at the end of the shredding step, generating and maintaining the vacuum inside the treatment chamber (4) for a first time interval (T1);
at the end of the first time interval (T1), interrupting the generation of the vacuum and feeding vapour under pressure into the treatment chamber (4) for a second time interval (T2); and
at the end of the second time interval (T2), interrupting the feeding of vapour and generating and maintaining again the vacuum inside the treatment chamber (4) for a third time interval (T3),
wherein during the step of feeding vapour under pressure into the treatment chamber (4), the shredding of the waste by means of the shredding device (9) is continuously performed,
and wherein, in order to continuously perform the shredding of the waste by means of the shredding device (9), the containing body (3) is rotated, so that a pushing element (8), which projects from an inner wall of the containing body (3), pushes the waste arranged inside the treatment chamber (4) from the bottom to the top.

17. A method according to claim 16, wherein the steps of feeding vapour under pressure into the treatment chamber (4) for the second time interval (T2) and of generating the vacuum inside the treatment chamber (4) for the third time interval (T3) are cyclically repeated in this order and for a predetermined number of times.

18. A method according to claim 16 or 17, wherein, during the step of generating the vacuum inside the treatment chamber (4), the shredding of the waste by means of the shredding device (9) is continuously performed.

19. A method according to any of the claims from 16 to 18 and comprising the further steps of:
discharging the gas sucked from the treatment chamber (4) into the atmosphere, by passing through at least one bacteriological filter (24), while the vacuum is generated for the first time interval (T1); and
discharging the gas sucked from the treatment chamber (4) into the atmosphere, causing it to flow through at least one burner (25), while the vacuum is generated for the third time interval (T3).

20. A method according to any of the claims from 16 to 19 and comprising the further step of continuously heating a lateral cylindrical wall of the containing body (3) during the generation of the vacuum inside the treatment chamber (4) and/or during the feeding of vapour into the treatment chamber (4).

## Patentansprüche

1. Maschine (1) zur Behandlung von gefährlichen medizinischen Abfällen; wobei die Maschine (1) umfasst:
einen zylindrischen Behälterkörper (3), der so montiert ist, dass er sich um eine erste Drehachse (5) dreht, und in dem eine Behandlungskammer (4) definiert ist;
eine erste Betätigungsvorrichtung (29), die geeignet ist, den Behälterkörper (3) um die erste Drehachse (5) zu drehen;
eine Einlass-/Auslassöffnung (6), die durch eine Seitenwand des Behälterkörpers (3) erhalten wird und mit einem mobilen Deckel (7) ausgestattet ist; und
eine Dampfzuführvorrichtung (19), die geeignet ist Dampf unter Druck in die Behandlungskammer (4) einzuspeisen;
wobei die Maschine (1) **dadurch gekennzeichnet ist, dass** sie umfasst:
mindestens ein Schiebeelement (8), das innerhalb der Behandlungskammer (4) angeordnet ist, sich von einer inneren Wand des Behälterkörpers (3) erstreckt und in dem Behälterkörper (3) integriert ist, um sich so zusammen mit dem Behälterkörper (3) um die erste Drehachse (5) zu drehen, um die Abfälle, die im Inneren der Behandlungskammer (4) angeordnet sind, von unten nach oben zu schieben;
eine Zerkleinerungsvorrichtung (9), die im Inneren der Behandlungskammer (4) in einer festen Position angeordnet ist, so dass sich der Behälterkörper (3) um die Zerkleinerungsvorrichtung (9) dreht;
eine Fördervorrichtung (10), die im Inneren der Behandlungskammer (4) und über der Zerkleinerungsvorrichtung (9) in einer festen Position angeordnet ist, so dass sich der Behälterkörper (3) um die Fördervorrichtung (10) dreht; und
eine Vakuumvorrichtung (21), die pneumatisch mit der Behandlungskammer (4) verbunden ist, um so im Inneren der Behandlungskammer (4) ein Vakuum zu erzeugen.

2. Maschine (1) gemäß Anspruch 1, die umfasst: einen mechanischen Filter (23), der an eine Auslassöffnung (22) der Behandlungskammer (4) gekoppelt ist, die mit der Vakuumvorrichtung (21) kommuniziert.

3. Maschine (1) gemäß Anspruch 1 oder 2, die umfasst: mindestens einen bakteriologischen Filter (24), der mit einem Auslass der Vakuumvorrichtung (21) verbunden werden kann, der das aus der Behandlungskammer (4) abgesaugte Gas in die Atmosphäre abführt.

4. Maschine (1) gemäß Anspruch 3, wobei die Dampfzufuhrvorrichtung (19) mit dem bakteriologischen Filter (24) verbunden sein kann, um den bakteriologischen Filter (24) zu sterilisieren.

5. Maschine (1) gemäß einem der vorangehenden Ansprüche, die umfasst: mindestens einen Brenner (25), der mit einem Auslass der Vakuumvorrichtung (21) verbunden werden kann, der das aus der Behandlungskammer (4) abgesaugte Gas in die Atmosphäre abführt, und geeignet ist das Gas für eine vorbestimmte Zeitspanne auf eine vorbestimmte Temperatur zu erhitzen.

6. Maschine (1) gemäß einem der vorangehenden Ansprüche, wobei die Fördervorrichtung eine "Trichterform" aufweist, die auf die Zerkleinerungsvorrichtung (9) konvergiert und eine flache Rutsche (11) umfasst, die geneigt angeordnet ist und nahe bei der Zerkleinerungsvorrichtung (9) endet.

7. Maschine (1) gemäß Anspruch 6, wobei die Fördervorrichtung (10) umfasst:
ein Presselement (12), das montiert ist, um entlang der Rutsche (11) zu gleiten; und
eine zweite Betätigungsvorrichtung (13), die mechanisch mit dem Presselement (12) verbunden ist, um zu bewirken, dass sich das Presselement (12) mit einer gradlinigen Rückbewegung parallel zu der Rutsche (11) bewegt.

8. Maschine (1) gemäß einem der vorangehenden Ansprüche, wobei die Zerkleinerungsvorrichtung (9) umfasst:
eine einzelne Zerkleinerungstrommel (14), die montiert ist, um sich um eine zweite Drehachse (15) zu drehen, die parallel zu der ersten Drehachse (5) verläuft, und mit Klingen ausgestattet ist, die sich von der Peripherie der Zerkleinerungstrommel (14) erstrecken;
eine dritte Betätigungsvorrichtung (30), die geeignet ist die Zerkleinerungstrommel (14) um die zweite Drehachse (15) zu drehen; und
einen festen Gegenkörper (16), der über eine gekrümmte Gegenwand (17) verfügt, der mit der Zerkleinerungstrommel (14) zusammenarbeitet und um einen Teil der Zerkleinerungstrommel (14) angeordnet ist.

9. Maschine (1) gemäß Anspruch 8, wobei der Gegenkörper (16) in seinem Querschnitt über eine "Kommaform" verfügt und die gekrümmte Gegenwand (17) auf der Seite, die der Zerkleinerungstrommel (14) gegenüberliegt, und auf der entgegengesetzten Seite, eine gekrümmte zugehörige Wand (18), die unten mit der Gegenwand (17) verbunden ist aufweist und zusammen mit einer Innenwand des Behälterkörpers (3), einen aufsteigenden Kanal definiert, entlang dem die Abfälle, aufgrund der Aktion des Schiebeelements (8), von unten nach oben geschoben werden.

10. Maschine (1) gemäß einem der vorangehenden Ansprüche, wobei eine zylindrische Seitenwand des Behälterkörpers (3) beheizbar ist.

11. Maschine (1) gemäß Anspruch 10, wobei die zylindrische Seitenwand des Behälterkörpers (3) einen Hohlraum (26) aufweist, in den Dampf eingespeist werden kann, der von der Dampfzuführvorrichtung (19) stammt.

12. Maschine (1) gemäß einem der vorangehenden Ansprüche, die eine Waschwasserzuführvorrichtung (31) umfasst, die geeignet ist Waschwasser in die Behandlungskammer (4) einzuspeisen.

13. Maschine (1) gemäß einem der vorangehenden Ansprüche, die umfasst:
zwei kreisförmige feste Flansche (27), die koaxial zu der ersten Drehachse (5) und auf gegenüberliegenden Seiten der Behandlungskammer (4) angeordnet sind, um zwei Endverschlüsse der Behandlungskammer (4) zu definieren, um zwei entsprechende ringförmige Lager (28) des Behälterkörpers (3), die entlang der Außenkante der Flansche (27) montiert sind, und die Zerkleinerungsvorrichtung (9) und die Fördervorrichtung (10) zu unterstützen.

14. Maschine (1) gemäß Anspruch 13, die umfasst: einen Rahmen (2), der auf dem Boden ruht, die zwei festen Flansche (27) starr unterstützt und unter dem Behälterkörper (3) einen Entladungsbereich definiert, der geeignet ist einen Abfallbehälter (33) für die behandelten Abfälle zu beherbergen, die durch Herabsinken durch die Auslass-/Einlassöffnung (6), die in einer unteren Position angeordnet ist, in den Abfallbehälter (33) fallen.

15. Maschine (1) gemäß einem der vorangehenden Ansprüche, die umfasst: ein Ladeband (34), das neben dem Behälterkörper (3) angeordnet ist und geeignet ist die zu behandelnden Abfälle über den Behälterkörper (3) zu heben, um so zu bewirken, dass sie durch Herabsinken durch die Einlass-/Auslassöffnung (6), die in einer oberen Position angeordnet ist, in die Behandlungskammer (4) fallen.

16. Verfahren zur Behandlung von gefährlichen medizinischen Abfällen; wobei das Verfahren die folgenden Schritte umfasst:
Einspeisung der Abfälle in eine Behandlungskammer (4), die im Inneren eines zylindrischen Behälterkörpers (3) definiert wird, die so montiert ist, dass sie sich um eine Drehachse (5) dreht; und
Zerkleinerung der Abfälle durch eine Zerkleinerungsvorrichtung (9), die im Inneren der Behandlungskammer (4) angeordnet ist;
dass das Verfahren **dadurch gekennzeichnet ist, dass** es die folgenden weiteren Schritte umfasst:
am Ende des Zerkleinerungsschritts, Erzeugung und Aufrechterhaltung des Vakuums im Inneren der Behandlungskammer (4) über eine erste Zeitspanne (T1);
am Ende der ersten Zeitspanne (T1), Unterbrechung der Erzeugung des Vakuums und Einspeisung von Dampf unter Druck in die Behandlungskammer (4) über eine zweite Zeitspanne (T2); und
am Ende der zweiten Zeitspanne (T2), Unterbrechung der Einspeisung von Dampf und erneute Erzeugung und Aufrechterhaltung des Vakuums im Inneren der Behandlungskammer (4) über eine dritte Zeitspanne (T3),
wobei während des Schritts zur Einspeisung von Dampf unter Druck in die Behandlungskammer (4) die Zerkleinerung der Abfälle durch die Zerkleinerungsvorrichtung (9) kontinuierlich durchgeführt wird,
und wobei, um die Zerkleinerung der Abfälle durch die Zerkleinerungsvorrichtung (9) kontinuierlich durchzuführen, der Behälterkörper (3) gedreht wird, so dass ein Schiebeelement (8), das sich von einer Innenwand des Behälterkörpers (3) erstreckt, die Abfälle, die im Inneren der Behandlungskammer (4) angeordnet sind, von unten nach oben schiebt.

17. Verfahren gemäß Anspruch 16, wobei die Schritte zur Einspeisung von Dampf unter Druck in die Behandlungskammer (4) über die zweite Zeitspanne (T2) und zur Erzeugung des Vakuums im Inneren der Behandlungskammer (4) über die dritte Zeitspanne (T3) in dieser Reihenfolge und mit einer vorbestimmten Häufigkeit zyklisch wiederholt werden.

18. Verfahren gemäß Anspruch 16 oder 17, wobei, während des Schrittes zur Erzeugung des Vakuums im Inneren der Behandlungskammer (4), die Zerkleinerung der Abfälle durch die Zerkleinerungsvorrichtung (9) kontinuierlich durchgeführt wird.

19. Verfahren gemäß einem der Ansprüche 16 bis 18, das die folgenden weiteren Schritte umfasst:
Abführung des aus der Behandlungskammer (4) abgesaugten Gases in die Atmosphäre, durch Passieren durch mindestens einen bakteriologischen Filter (24), während das Vakuum über die erste Zeitspanne (T1) erzeugt wird; und
Abführung des aus der Behandlungskammer (4) abgesaugten Gases in die Atmosphäre, wobei es veranlasst wird durch mindestens einen Brenner (25) zu strömen, während das Vakuum über die dritte Zeitspanne (T3) erzeugt wird.

20. Verfahren gemäß einem der Ansprüche 16 bis 19, das umfasst: den weiteren Schritt zur kontinuierlichen Erhitzung einer zylindrischen Seitenwand des Behälterkörpers (3) während der Erzeugung des Vakuums im Inneren der Behandlungskammer (4) und/oder während der Einspeisung von Dampf in die Behandlungskammer (4).

## Revendications

1. Machine (1) de traitement des déchets médicaux dangereux ; la machine (1) comprenant :
un corps de contenance cylindrique (3), monté pour tourner autour d'un premier axe de rotation (5) et à l'intérieur duquel une chambre de traitement (4) est définie ;
un premier dispositif d'actionnement (29), apte à faire tourner le corps de contenance (3) autour du premier axe de rotation (5) ;
une ouverture d'entrée/sortie (6), créée dans une paroi latérale du corps de contenance (3) et équipée d'un rabat mobile (7) ; et
un dispositif d'alimentation en vapeur (19), apte à alimenter en vapeur sous pression la chambre de traitement (4) ;
la machine (1) étant **caractérisée en ce qu'**elle comprend :
au moins un élément de poussée (8), disposé à l'intérieur de la chambre de traitement (4), dépassant d'une paroi interne du corps de contenance (3) et faisant partie intégrante du corps de contenance (3) de manière à pouvoir tourner autour du premier axe de rotation (5) avec le corps de contenance (3), afin de pousser les déchets se trouvant à l'intérieur de la chambre de traitement (4) de bas en haut ;
un dispositif de déchiquetage (9), installé à l'intérieur de la chambre de traitement (4) dans une position fixe de sorte que le corps de contenance (3) puisse tourner autour du dispositif de déchiquetage (9) ;
un dispositif de transport (10), installé à l'intérieur de la chambre de traitement (4) et au-dessus du dispositif de déchiquetage (9) dans une position fixe, de sorte que le corps de contenance (3) tourne autour du dispositif de transport (10) ; et
un dispositif à vide (21), relié de manière pneumatique à la chambre de traitement (4) afin de créer un vide à l'intérieur de la chambre de traitement (4).

2. Machine (1) selon la revendication 1 et comprenant un filtre mécanique (23), couplé à une ouverture de sortie (22) de la chambre de traitement (4) qui communique avec le dispositif à vide (21).

3. Machine (1) selon la revendication 1 ou 2 et comprenant au moins un filtre bactériologique (24) qui peut être relié à une sortie du dispositif à vide (21) qui dégage le gaz aspiré de la chambre de traitement (4) dans l'atmosphère.

4. Machine (1) selon la revendication 3, dans laquelle le dispositif d'alimentation en vapeur (19) peut être relié au filtre bactériologique (24) afin de stériliser le filtre bactériologique (24).

5. Machine (1) selon l'une quelconque des revendications précédentes, comprenant au moins un brûleur (25) qui peut être relié à une sortie du dispositif à vide (21), qui dégage le gaz aspiré de la chambre de traitement (4) dans l'atmosphère, et est apte à chauffer le gaz à une température prédéterminée pendant un intervalle de temps prédéterminé.

6. Machine (1) selon l'une quelconque des revendications précédentes, dans laquelle le dispositif de transport présente une forme d'« entonnoir » qui converge sur le dispositif de déchiquetage (9) et comprend une glissière plate (11), agencée de manière inclinée et dont les extrémités se trouvent à proximité du dispositif de déchiquetage (9).

7. Machine (1) selon la revendication 6, dans laquelle le dispositif de transport (10) comprend :
un élément de pression (12), monté pour coulisser le long de la glissière (11) ; et
un deuxième dispositif d'actionnement (13), relié mécaniquement à l'élément de pression (12) pour permettre à l'élément de pression (12) de se déplacer avec un mouvement de réciprocité linéaire parallèle à la glissière (11).

8. Machine (1) selon l'une quelconque des revendications précédentes, dans laquelle le dispositif de déchiquetage (9) comprend :
un tambour de déchiquetage unique (14), monté pour tourner autour d'un second axe de rotation (15), parallèle au premier axe de rotation (5), et équipé de lames qui dépassent de la périphérie du tambour de déchiquetage (14) ;
un troisième dispositif d'actionnement (30), apte à faire tourner le tambour de déchiquetage (14) autour du second axe de rotation (15) ; et
un corps de contenance fixe (16) possédant une paroi de contrebalance incurvée (17) coopérant avec le tambour de déchiquetage (14) et disposé autour d'une partie du tambour de déchiquetage (14).

9. Machine (1) selon la revendication 8, dans laquelle le corps de contenance (16) présente une forme de « virgule » dans sa section transversale et possède la paroi de contrebalance incurvée (17) sur le côté faisant face au tambour de déchiquetage (14) et, sur le côté opposé, une paroi d'accompagnement incurvée (18) qui, sur le côté inférieur, est reliée à la paroi de contrebalance incurvée (17) et définit, avec une paroi interne du corps de contenance (3), un canal ascendant le long duquel les déchets sont poussés de bas en haut sous l'effet de l'élément de poussée (8).

10. Machine (1) selon l'une quelconque des revendications précédentes, dans laquelle une paroi cylindrique latérale du corps de contenance (3) peut être chauffée.

11. Machine (1) selon la revendication 10, dans laquelle la paroi cylindrique latérale du corps de contenance (3) possède un espace creux (26) dans lequel la vapeur provenant du dispositif d'alimentation en vapeur (19) peut être alimentée.

12. Machine (1) selon l'une quelconque des revendications précédentes, comprenant un dispositif d'alimentation en eau de lavage (31), apte à alimenter en eau de lavage la chambre de traitement (4).

13. Machine (1) selon l'une quelconque des revendications précédentes, comprenant :
deux brides fixes (27) de forme circulaire, coaxiales au premier axe de rotation (5), disposées sur les côtés opposés de la chambre de traitement (4) afin de définir deux fermetures d'extrémité de la chambre de traitement (4), de supporter deux roulements annulaires correspondants (28) du corps de contenance (3), montés le long du bord extérieur des brides (27) et de supporter le dispositif de déchiquetage (9) et le dispositif de transport (10).

14. Machine (1) selon la revendication 13 et comprenant un cadre (2) qui repose sur le sol, supporte de manière rigide les deux brides fixes (27) et définit, sous le corps de contenance (3), une zone de dégagement, apte à loger un conteneur de dégagement (33) pour les déchets traités qui tombent, sous l'action de la gravité, dans le conteneur de dégagement (33), via l'ouverture d'entrée/sortie (6), disposée dans une position inférieure.

15. Machine (1) selon l'une quelconque des revendications précédentes, comprenant un transporteur de chargement (34), disposé à proximité du corps de contenance (3) et apte à soulever les déchets à traiter au-dessus du corps de contenance (3) pour les faire tomber, sous l'effet de la gravité, dans la chambre de traitement (4) et via l'ouverture d'entrée/sortie (6), disposée dans une position supérieure.

16. Procédé de traitement des déchets médicaux dangereux, le procédé comprenant les étapes suivantes :
alimenter en déchets la chambre de traitement (4), qui se trouve à l'intérieur d'un corps de contenance cylindrique (3), monté pour tourner autour d'un axe de rotation (5) ; et
déchiqueter les déchets à l'aide d'un dispositif de déchiquetage (9), disposé à l'intérieur de la chambre de traitement (4) ;
le procédé étant **caractérisé en ce qu'**il comprend les étapes supplémentaires suivantes :
à la fin de l'étape de déchiquetage, créer et maintenir le vide à l'intérieur de la chambre de traitement (4) pendant un premier intervalle (T1) ;
à la fin du premier intervalle (T1), interrompre la création du vide et alimenter en vapeur sous pression la chambre de traitement (4) pendant un deuxième intervalle (T2) ; et
à la fin du deuxième intervalle (T2), interrompre l'alimentation en vapeur et créer et maintenir à nouveau le vide à l'intérieur de la chambre de traitement (4) pendant un troisième intervalle (T3),
dans lequel, pendant l'étape d'alimentation en vapeur sous pression de la chambre de traitement (4), le déchiquetage des déchets à l'aide du dispositif de déchiquetage (9) est effectué de manière continue,
et dans lequel, afin de poursuivre le déchiquetage des déchets à l'aide du dispositif de déchiquetage (9), le corps de contenance (3) tourne de sorte qu'un élément de poussée (8), qui dépasse d'une paroi interne du corps de contenance (3), pousse les déchets disposés à l'intérieur de la chambre de traitement (4) de bas en haut.

17. Procédé selon la revendication 16, dans lequel les étapes d'alimentation en vapeur sous pression de la chambre de traitement (4) pendant le deuxième intervalle (T2) et de création de vide à l'intérieur de la chambre de traitement (4) pendant le troisième intervalle (T3) sont répétées de manière cyclique dans cet ordre et un nombre de fois prédéterminé.

18. Procédé selon la revendication 16 ou 17, dans lequel, pendant l'étape de création de vide à l'intérieur de la chambre de traitement (4), le déchiquetage des déchets à l'aide du dispositif de déchiquetage (9) est effectué de manière continue.

19. Procédé selon l'une quelconque des revendications 16 à 18 et comprenant les étapes supplémentaires suivantes :
dégager le gaz aspiré de la chambre de traitement (4) dans l'atmosphère en traversant au moins un filtre bactériologique (24) lorsque le vide est créé pendant le premier intervalle (T1) ; et
dégager le gaz aspiré de la chambre de traitement (4) dans l'atmosphère en le faisant circuler à travers au moins un brûleur (25), alors que le vide est créé pendant le troisième intervalle (T3).

20. Procédé selon l'une quelconque des revendications 16 à 19 et comprenant l'étape supplémentaire de chauffage en continu d'une paroi cylindrique latérale du corps de contenance (3) pendant la création du vide à l'intérieur de la chambre de traitement (4) et/ou pendant l'alimentation en vapeur de la chambre de traitement (4).
